(19) Europäisches Patentamt
European Patent Office
Office européen des brevets

(11) Veröffentlichungsnummer: **0 155 400**
**B1**

(12) # EUROPÄISCHE PATENTSCHRIFT

(45) Veröffentlichungstag der Patentschrift: **15.02.89**

(51) Int. Cl.⁴: **A 61 K 7/11**

(21) Anmeldenummer: **84116029.4**

(22) Anmeldetag: **21.12.84**

(54) **Mittel zur Festigung der Frisur und Pflege des Haares.**

(30) Priorität: **13.01.84 DE 3401037**

(43) Veröffentlichungstag der Anmeldung: **25.09.85 Patentblatt 85/39**

(45) Bekanntmachung des Hinweises auf die Patenterteilung: **15.02.89 Patentblatt 89/7**

(84) Benannte Vertragsstaaten: **AT BE CH DE FR GB IT LI LU NL SE**

(56) Entgegenhaltungen:
**FR-A-2 251 307**
**GB-A-1 584 127**
**GB-A-2 098 226**
**US-A-3 954 960**
**US-A-3 958 581**

**SEIFEN - ÖLE - FETTE - WACHSE, Jg. 99, Nr. 12, 1973, Seiten 333-337, Verlag für chemische Industrie H. ZIOLKOWSKI, Augsburg, DE; G.P. LAUHUS: "GAFQUAT - Copolymere, eine neue Generation kationaktiver Harze für die Haarkosmetik"**

**Die Akte enthält technische Angaben, die nach dem Eingang der Anmeldung eingereicht wurden und die nicht in dieser Patentschrift enthalten sind.**

(73) Patentinhaber: **Wella Aktiengesellschaft, Berliner Allee 65, D-6100 Darmstadt (DE)**

(72) Erfinder: **Gross, Paul, Forstweg 54, D-6100 Darmstadt (DE)**
Erfinder: **Wiegand, Udo, Rappmühlstrasse 29, D-6108 Weiterstadt 3 (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

EP 0 155 400 B1

LIBER, STOCKHOLM 1989

**Beschreibung**

Gegenstand der Erfindung ist ein Mittel zur Festigung der Frisur und Pflege des Haares, welches Tetraoxyethylenlaurylether und ein quaternisiertes Copolymerisat aus Vinylpyrrolidon mit Dimethylaminoethylmethacrylat (80 : 20) enthält.

Mittel zur Festigung der Frisur und Pflege des Haares bestehen üblicherweise aus Lösungen von filmbildenden natürlichen oder synthetischen Polymeren. Als natürliche Polymere kommen beispielsweise Schellack, Alginate, Gelatine, Pektine, Chitosansalze und Cellulosederivate in Betracht. Von den synthetischen Polymeren finden zum Beispiel Polyvinylpyrrolidon, Polyvinylacetat, Polyacrylverbindungen wie Acrylsäure- oder Methacrylsäurepolymerisate, basische Polymerisate von Estern aus diesen beiden Säuren mit Aminoalkoholen bzw. die Salze oder Quaternisierungsprodukte dieser basischen Polymerisate, Polyacrylnitril sowie Copolymerisate aus derartigen Verbindungen, beispielsweise Polyvinylpyrrolidon-Vinylacetat, Verwendung.

Aus der US-A-3 954 960 sind Mittel zur Festigung und Pflege der Haare bekannt, die neben einem quaternisierten Copolymerisat aus Vinylpyrrolidon und Dimethylaminoethylmethacrylat (80 : 20) einen mit 3 beziehungsweise 10 Mol Ethylenoxid oxethylierten Isostearylalkohol enthalten.

Weiterhin wird in der GB-A-2 098 226 ein Haarbehandlungsmittel auf der Basis eines quaternisierten Copolymers aus Vinylpyrrolidon und Dimethylaminoethylmethacrylat (Gafquat® 755) sowie eines mit 12 Mol Ethylenoxid oxethylierten Cetyl-stearyl-alkohols beschrieben.

Ebenfalls ist aus der US-A-3 958 581 bekannt, in Haarpflegemitteln ein quaternisiertes Copolymerisat aus Vinylpyrrolidon und Dimethylaminoethylmethacrylat (Gafquat ® 755) in Kombination mit ejnem mit 20 Mol Ethylenoxid oxethylierten Lanolinalkohol einzusetzen.

Bezüglich der haarfestigenden und insbesondere haarkonditionierenden Eigenschaften sind die vorstehend genannten Mittel jedoch alle nicht völlig befriedigend.

Weiterhin enthalten Mittel zur Pflege des Haares und Festigung der Frisur zwecks Verbesserung der Naßkämmbarkeit und des Griffes, insbesondere des geschädigten Haares, häufig monomere quaternäre Ammoniumverbindungen wie zum Beispiel Alkyltrimethylammoniumchloride, Dialkyldimethylammoniumchloride, Alkyldimethylbenzylammoniumchloride und Alkylpyridiniumchloride.

Derartige Zusätze quaternärer Ammoniumverbindungen verschlechtern jedoch die physiologische Verträglichkeit solcher Präparate, insbesondere die Augenverträglichkeit.

In der GB-A-1 584 127 wird ferner ein Konditionierungsshampoo beschrieben, das eine Kombination eines kationischen Polymers, unter anderem eines Poly-(aminoethyl-acrylat-hydroxypropyl-acrylat)-phospats, mit einem nicht-ionischen Tensid, beispielsweise einen Tetraoxyethylenlaurylether, enthält.

Dieses Konditionierungsshampoo besitzt zwar ausreichende haarkonditionierende Eigenschaften, jedoch hat es trotz seines relativ hohen Gehalts an kationischen Polymeren von bis zu 10 Gewichtsprozent keine befriedigende haarfestigende Wirkung.

Demgegenüber wurde nun gefunden, daß ein Mittel zur Festigung der Frisur und Pflege des Haares auf der Basis einer wäßrigen, alkoholischen oder wäßrig-alkoholischen Lösung eines quaternisierten Copolymerisates aus Vinylpyrrolidon und Dimethylaminoethylmethacrylat (80 : 20) sowie eines oxethylierten Fettalkohols und gegebenenfalls Treibmitteln und üblichen Zusätzen, dadurch gekennzeichnet, daß es

a) 0,1 bis 10,0 Gew. % des quaternisierten Copolymerisates aus Vinylpyrrolidon und Dimethylaminoethylmethacrylat (80 : 20) und
b) als oxethylierten Fettalkohol 0,1 bis 5,0 Gew. % Tetraoxyethylenlaurylether

enthält, eine überraschende und ausgezeichnete Verbesserung sowohl der Kämmbarkeit des Haares als auch der Festigung der Frisur bewirkt.

Das unter a) genannte Copolymerisat aus Vinylpyrrolidon und Dimethylaminoethylmethacrylat soll vorzugsweise mit Dimethylsulfat oder Diethylsulfat quaternisiert sein. Als quaternisiertes Copolymerisat aus Vinylpyrrolidon und Dimethylaminoethylmethacrylat (80 : 20) sind besonders die Handesprodukte GAFQUAT® 734 und GAFQUAT® 755 der Firma GAF Corporation, New York geeignet. Diese weisen die folgende Formel auf:

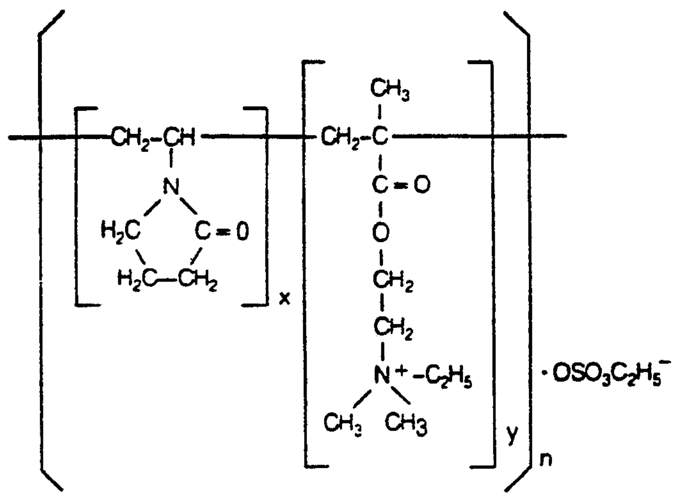

(Verhältnis x:y= 80:20)

Das mittlere Molekulargewicht beträgt bei GAFQUAT® 734 etwa 100 000 und bei GAFQUAT® 755 etwa 1000 000 (g/Mol).

Tetraoxyethylenlaurylether der Formel

$$CH_3(CH_2)_{10}CH_2(OCH_2CH_2)_4OH$$

ist der Tetraoxyethylenether des Laurylalkohols.

In dem erfindungsgemäßen Mittel soll der Tetraoxyethylenlaurylether in einer Menge von 0,1 bis 5 Gew. %, vorzugsweise in einer Menge von 0,5 bis 2 Gew. %, enthalten sein. Der Gehalt an dem quaternisierten Copolymerisat aus Vinylpyrrolidon und Dimethylaminoethylmethacrylat beträgt 0,1 bis 10 Gew. %, vorzugsweise 1 bis 3,5 Gew. %. Ethylalkohol und Isopropylalkohol oder Gemische dieser beiden Alkohole können in dem erfindungsgemäßen Mittel in einer Menge von 0 bis 99,8 Gew. % enthalten sein. Selbstverständlich können auch übliche haarkosmetische Zusätze wie beispielsweise Parfümöle, Kräuterextrakte, bakterizide oder fungizide Stoffe, Antischuppenmittel, Lösungsvermittler für Parfümöle, natürliche oder synthetische Harze wie Chitosansalze, Polyvinylpyrrolidon, Polyvinylpyrrolidon-Vinylacetat-Copolymere in dem erfindungsgemäßen Mittel enthalten sein, soweit solche Zusätze nützlich und zweckmäßig erscheinen. Weiterhin können auch Anfärbefarbstoffe zur Anfärbung des Präparates oder direkt auf das Haar aufziehende Farbstoffe zur gleichzeitigen Tönung des Haares enthalten sein.

Von letzteren Farbstoffen, die einzeln oder in Mischung vorliegen können, seien beispielsweise die folgenden Klassen erwähnt:

Aromatische Nitrofarbstoffe, z. B. 1,4-Diamino-2-nitrobenzol, Azofarbstoffe, z. B. Acid Brown 4 (C.I. Nr. 14 805), Anthrachinonfarbstoffe, z. B. Disperse Violet 4 (C.I. Nr. 61 105) und Triphenylmethanfarbstoffe, z. B. Basie Violet 1 (C.I. Nr. 42 535), wobei die Farbstoffe dieser Klassen je nach der Art ihrer Substituenten sauren, nichtionogenen oder basischen Charakter haben können. Ihre Gesamtkonzentration beträgt üblicherweise etwa 0,05 bis 2,0 Gew. %.

Die erfindungsgemäßen Mittel können auch unter Zusatz eines Treibmittels in einem Druckbehälter abgefüllt vorliegen, wobei das Präparat als Schaum austritt und mittels eines mit einer Auftragedüse versehenen Ventils leicht dosiert und bequem auf dem Haar verteilt werden kann. Als Treibmittel sind beispielsweise leicht flüchtige Fluorchlorkohlenwasserstoffe, wie z. B. Difluordichlormethan, Trichlormonofluormethan, Tetrafluordichlorethan oder niedere Alkane, wie z. B. n-Butan, i-Butan und Propan oder auch Dimethylether sowie ferner bei der in Betracht kommenden Drücken gasförmig vorliegende Treibmittel, wie $N_2$, $N_2O$ und $CO_2$, geeignet. Die Treibmittel sind in diesen Mitteln zweckmäßigerweise in einer Menge von etwa 2 bis 10 Gew. % enthalten.

Das erfindungsgemäße Mittel wird, üblicherweise nach der Haarwäsche, in dem handtuchtrockenen Haar in einer Menge von etwa 5 bis 20 g je nach Haarfülle verteilt. Anschließend wird das Haar durchgekämmt und in üblicher Weise entweder unmittelbar geföhnt oder zunächst auf Wasserwellwickler gewickelt und sodann getrocknet.

Die beobachtete ausgezeichnete Verbesserung der Kämmbarkeit des Haares und der Festigung der Frisur ist überraschend und nur synergistisch erklärbar, da der Tetraoxyethylenlaurylether oder das quaternisierte Copolymerisat aus Vinylpyrrolidon und Dimethylaminoethylmethacrylat in entsprechender Lösung allein auf das Haar aufgebracht (siehe Testbeispiele C und D) nur eine mäßige und unbefriedigende Verbesserung der genannten Kriterien bewirkt.

Weiterhin wurde durch die nachstehenden Testbeispiele E, F, G und H nachgewiesen, daß andere Fettalkoholpolyoxyethylenether als Tetraoxyethylenlaurylether die für eine Verwendung in den

erfindungsgemäßen Mitteln erforderliche synergistische Wirkung nicht aufweisen.

Bei dem erfindungsgemäßen Mittel zur Festigung der Frisur und Pflege des Haares kann auf Grund seiner ausgezeichneten kämmbarkeitsverbessernden Wirkung ganz auf die Verwendung quaternärer Ammoniumverbindungen verzichtet werden, es sei denn, man setzt sie dem Mittel in geringer Konzentration von etwa bis zu 0,2 Gew. % als Konservierungsmittel zur Erzielung bakterizider oder fungizider Eigenschaften zu.

Die nachstehenden Beispiele sollen den Gegenstand der Erfindung näher erläutern.

**Beispiele**

**Beispiel 1**

Mittel in Form einer wäßrigen Lösung

| | |
|---|---|
| 1,2 g | Tetraoxyethylenlaurylether |
| 2,5 g | Copolymerisat aus Vinylpyrrolidon und Dimethylaminoethylmethacrylat im Verhältnis 80 : 20, quaternisiert mit Diethylsulfat |
| 96,3 g | Wasser, vollentsalzt |
| 100,0 g | |

**Beispiel 2**

Mittel in Form einer wäßrig-alkoholischen Lösung

| | |
|---|---|
| 0,7 g | Tetraoxyethylenlaurylether |
| 2,0 g | Copolymerisat aus Vinylpyrrolidon und Dimethylaminoethylmethacrylat im Verhältnis 80 : 20, quaternisiert mit Diethylsulfat |
| 20,0 g | Ethanol |
| 77,3 g | Wasser, vollentsalzt |
| 100,0 g | |

**Beispiel 3**

Mittel in Form einer Druckgaspackung

| | |
|---|---|
| 1,5 g | Tetraoxyethylenlaurylether |
| 2,5 g | Copolymerisat aus Vinylpyrrolidon und Dimethylaminoethylenmethacrylat im Verhältnis 80 : 20, quaternisiert mit Diethylsulfat |
| 10,0 g | Isopropanol |
| 86,0 g | Wasser, vollentsalzt |
| 100,0 g | |

Abfüllung: 96,0 g Flüssigkeit der vorstehenden Zusammensetzung
4,0 g Propan

**Beispiel 4**

Mittel in Form einer Druckgaspackung

| | |
|---|---|
| 1,4 g | Tetraoxyethylenlaurylether |
| 2,0 g | Copolymerisat aus Vinylpyrrolidon und Dimethylaminoethylmethacrylat im Verhältnis 80 : 20, quaternisiert mit Diethylsulfat |
| 8,0 g | Ethanol |
| 88,6 g | Wasser, vollentsalzt |
| 100,0 g | |

Abfüllung: 96,0 g Flüssigkeit der vorstehenden Zusammensetzung
2,0 g Propan
1,6 g Butan
0,4 g Dimethylether

**Beispiel 5**

Mittel in Form einer wäßrig-alkoholischen Lösung

| | |
|---|---|
| 1,20 g | Tetraoxyethylenlaurylether |
| 2,50 g | Copolymerisat aus Vinylpyrrolidon und Dimethylaminoethylmethacrylat im Verhältnis 80 : 20, quaternisiert mit Diethylsulfat |
| 0,50 g | Polyvinylpyrrolidon |
| 0,05 g | Basie Violet 1 (C.I. Nr. 42 535) |
| 5,00 g | Ethanol |
| 90,75 g | Wasser, vollentsalzt |
| 100,00 g | |

Alle in der vorliegenden Anmeldung angegebenen Prozentzahlen stellen Gewichtsprozente dar.

**Versuchsbeispiele**

**Testbeispiel A**

Nach einer vorangegangenen Haarwäsche wurde das handtuchtrockene Haar von 20 Versuchspersonen mit einem Mittel gemäß Beispiel 1 behandelt. Zehn der Versuchspersonen hatten normales Haar. Die restlichen zehn Personen hatten geschädigtes bis stark geschädigtes Haar. Um eindeutige Ergebnisse zu erhalten und von Versuchsperson zu Versuchsperson abweichende Haarqualitäten auszuschalten, wurde das Haar in der Mitte gescheitelt und auf die eine Hälfte des Haares, in Abhängigkeit von der Haarfülle, 2,5 bis 6 g des Mittels aufgetragen, während die andere Hälfte unbehandelt blieb. Anschließend wurde jede Hälfte des Haares für sich durchgekämmt, auf Wasserwellwickler gewickelt und getrocknet. Schließlich wurden die Wickler entfernt und das Haar frisiert. So konnte die Wirkung des Präparates von einer friseur-fachlichen Expertengruppe sicher beurteilt werden. Eine Bewertung erfolgte nach dem Schema für die zusammengefaßten kriterien der Naßkämmbarkeit, Haltbarkeit der Frisur, Lockigkeit, Glanz, Griff und der statischen Aufladung des Haares:

Benotung: 1 = sehr gut
2 = gut
3 = ausreichend
4 = mangelhaft

Das Ergebnis des Versuches für die so behandelte Haarhälfte im Vergleich zur unbehandelten Hälfte zeigt die nachstehende Tabelle 1.

**Tabelle 1**

| | Note 1 | Note 2 | Note 3 | Note 4 |
|---|---|---|---|---|
| Anzahl der Versuchspersonen | 16 | 3 | 1 | 0 |

**Testbeispiel B**

Die Haare einer weiteren Gruppe von 18 Versuchspersonen wurden mit einem Mittel gemäß Beispiel 3 in gleicher Weise wie im Testbeispiel A behandelt. Zehn Personen der Gruppe hatten geschädigtes bis stark geschädigtes Haar. Die restlichen 8 Personen hatten normales Haar. Die Ergebnisse dieses Tests sind in der nachstehenden Tabelle 2 zusammengefaßt.

**Tabelle 2**

| | Note 1 | Note 2 | Note 3 | Note 4 |
|---|---|---|---|---|
| Anzahl der Versuchspresonen | 13 | 4 | 1 | 0 |

**Testbeispiel C**

Zur Überprüfung des synergistischen Effektes wurden die Haare einer weiteren Testgruppe von 14 Personen, wie im Testbeispiel A beschrieben, halbseitig mit einer Zusammensetzung gemäß Beispiel 1 behandelt, jedoch wurde der Tetraoxyethylenlaurylether durch die entsprechende Menge an Wasser ersetzt. Die andere Hälfte des Haares blieb unbehandelt. 5 Versuchspersonen hatten normales Haar und 9 Personen hatten geschädigtes Haar. Das Versuchsergebnis ist in Tabelle 3 wiedergegeben.

**Tabelle 3**

| | Note 1 | Note 2 | Note 3 | Note 4 |
|---|---|---|---|---|
| Anzahl der Versuchspersonen | 0 | 3 | 6 | 5 |

**Testbeispiel D**

Zur weiteren Überprüfung des synergistischen Effektes wurde eine vierte Testgruppe von 13 Personen, von denen sechs Personen normales und 7 Personen stark geschädigtes Haar hatten, in gleicher Weise wie in Testbeispiel A beschrieben, mit einer Zusammensetzung gemäß Beispiel 1 halbseitig behandelt, jedoch enthielt das Mittel kein quaternisiertes Copolymerisat aus Vinylpyrrolidon und Dimethylaminoethylmethacrylat (80 : 20). In der Zusammensetzung nach Beispiel 1 wurde dabei das quaternisierte Copolymerisat durch den entsprechenden Gewichtsanteil an Wasser ersetzt. Die zweite Hälfte des Haares blieb unbehandelt. Das Ergebnis des Versuches ist in der nachstehenden Tabelle 4 wiedergegeben.

**Tabelle 4**

| | Note 1 | Note 2 | Note 3 | Note 4 |
|---|---|---|---|---|
| Anzahl der Versuchspersonen | 0 | 4 | 4 | 5 |

Ein Vergleich der Tabellen 1 bzw. 2 sowohl mit der Tabelle 3 als auch mit der Tabelle 4 zeigt deutlich, daß die in den erfindungsgemäßen Mitteln enthaltene Kombination eine synergistische Wirkung im Vergleich zur Wirkung der Einzelkomponenten besitzt.

**Testbeispiel E**

Die Haare einer Testgruppe von 10 Personen mit geschädigtem bis stark geschädigtem Haar wurden analog Testbeispiel A halbseitig mit einer Präparatezusammensetzung nach Beispiel 1 behandelt, in dem jedoch der Tetraoxyethylenlaurylether mengengleich durch Trioxyethylenstearylether ersetzt worden war. Die andere Seite des Haares blieb unbehandelt. Das Versuchsergebnis zeigt die nachstehende Tabelle 5.

**Tabelle 5**

| | Note 1 | Note 2 | Note 3 | Note 4 |
|---|---|---|---|---|
| Anzahl der Versuchsperson | 0 | 1 | 8 | 1 |

**Testbeispiel F**

Es wurden die Haare einer Gruppe von 10 Versuchspersonen mit geschädigtem bis stark geschädigtem Haar analog Testbeispiel A halbseitig mit einer Präparatezusammensetzung nach Beispiel 1 behandelt, in dem

jedoch der Tetraoxyethylenlaurylether mengengleich durch Isostearylalkohol, ethoxyliert mit 10 Mol Ethylenoxid, ersetztworden war. Die andere Seite des Haares blieb unbehandelt. Das Ergebnis des Versuches zeigt die nachstehende Tabelle 6.

**Tabelle 6**

| | Note 1 | Note 2 | Note 3 | Note 4 |
|---|---|---|---|---|
| Anzahl der Versuchspersonen | 0 | 1 | 5 | 4 |

Die vorstehenden Testbeispiele E und F zeigen, daß unter den Polyoxyethylenlaurylethern Tetraoxyethylenlaurylether einen deutlidensynergistischen Effekt aufweist.

**Testbeispiel G**

Eine weitere Gruppe von 10 Versuchspersonen mit geschädigtem bis stark geschädigtem Haar wurde analog Testbeispiel A halbseitig mit einer Zusammensetzung gemäß Beispiel 1 behandelt, in dem jedoch der Tetraoxyethylenlaurylether mengengleich durch Trioxyethylenlaurylether ersetzt worden war. Die andere Seite des Haares blieb unbehandelt. Das Ergebnis des Versuches zeigt die nachstehende Tabelle 7.

**Tabelle 7**

| | Note 1 | Note 2 | Note 3 | Note 4 |
|---|---|---|---|---|
| Anzahl der Versuchspersonen | 0 | 3 | 6 | 1 |

**Testbeispiel H**

Ebenso wurde eine Testgruppe von 10 Personen mit geschädigtem bis stark geschädigtem Haar analog Testbeispiel A halbseitig mit einer Zusammensetzung gemäß Beispiel 1 behandelt, in dem jedoch der Tetraoxyethylenlaurylether mengengleich durch Pentaoxyethylenlaurylether ersetzt worden war. Die andere Seite des Haares blieb unbehandelt.

Das Ergebnis zeigt die nachstehende Tabelle 8.

**Tabelle 8**

| | Note 1 | Note 2 | Note 3 | Note 4 |
|---|---|---|---|---|
| Anzahl der Versuchspersonen | 0 | 3 | 5 | 2 |

Die Resultate der vorstehenden Versuche zeigen, daß andere Fettalkoholpolyoxyethylenether als Tetraoxyethylenlaurylether für eine erfindungsgemäüe Verwendung ungeeignet sind.

Die überraschende und ausgezeichnete synergistische Verbesserung der haarkonditionierenden Eigenschaften und die sehr gute Festigung der Frisur, insbesondere bei stark geschädigtem Haar, ist nach den beschriebenen Versuchsergebnissen auf die erfindungsgemäße Kombination von Tetraoxyethylenlaurylether mit einem quaternisierten Copolymerisat aus Vinylpyrrolidon und Dimethylaminoethylmethacrylat (80 : 20) beschränkt und ist mit anderen Fettalkoholpolyoxyethylenethern nicht zu erzielen.

**Patentansprüche**

1. Mittel zur Festigung der Frisur und Pflege des Haares auf der Basis einer wäßrigen, alkoholischen oder wäßrig-alkoholischen Lösung eines quaternisierten Copolymerisates aus Vinylpyrrolidon und Dimethylaminoethylmethacrylat (80 : 20) sowie eines oxethylierten Fettalkohols und gegebenenfalls Treibmitteln und üblichen Zusätzen, dadurch gekennzeichnet, daß es

a) 0,1 bis 10,0 Gew. % des quaternisierten Copolymerisates aus Vinylpyrrolidon und Dimethylaminoethylmethacrylat (80 : 20) und
b) als oxethylierten Fettalkohol 0,1 bis 5,0 Gew. % Tetraoxyethylenlaurylether

enthält.

2. Mittel nach Anspruch 1, dadurch gekennzeichnet, daß es 1 bis 3,5 Gew. % eines mit Dimethyl- oder

Diethylsulfat quaternisierten Copolymerisats aus Vinylpyrrolidon und Dimethylaminoethylmethacrylat (80 : 20) enthält.

3. Mittel nach Anspruch 1, dadurch gekennzeichnet, daß es 0,5 bis 2,0 Gew. % Tetraoxyethylenlaurylether enthält.

4. Mittel nach Anspruch 1, dadurch gekennzeichnet, daß es zur gleichzeitigen Tönung des Haares 0,05 bis 2,0 Gew. % eines direkt auf das Haar aufziehenden Farbstoffs enthält.

5. Mittel nach Anspruch 4, dadurch gekennzeichnet, daß der direkt auf das Haar aufziehende Farbstoff ausgewählt ist aus aromatischen Nitrofarbstoffen, Azofarbstoffen, Anthrachinonfarbstoffen und Triphenylmethanfarbstoffen.

6. Mittel nach den Ansprüchen 1 bis 5, dadurch gekennzeichnet, daß es 2 bis 10 Gew. % eines Treibmittels enthält, das ausgewählt ist aus n-Butan, i-Butan, Propan, Difluordichlormethan, Trichlormonofluormethan, Tetrafluordichlorethan, Dimethylether, $N_2$, $N_2O$ und $CO_2$.

## Claims

1. Composition for fixing a hairstyle and for haircare based on an aqueous, alcoholic or aqueous-alcoholic solution of a quaternised copolymer of vinyl pyrrolidone and dimethylaminoethyl methacrylate (80 : 20) as well as an ethoxylated fatty alcohol and where applicable propellants and usual additives, characterized in that it contains:

a) 0.1 to 10.0 % by weight of the quaternised copolymer of vinyl pyrrolidone and dimethylaminoethyl methacrylate (80 : 20) and

b) 0.1 to 5.0 % by weight of tetraoxyethylene lauryl ether as an ethoxylated fatty alcohol.

2. Composition according to claim 1, characterized in that it contains 1 to 3.5 % by weight of a copolymer of vinyl pyrrolidone and dimethylaminoethyl methacrylate (80 : 20) quarternized with dimethyl or diethylsulphate.

3. Composition according to claim 1, characterized in that it contains 0.5 to 2.0 % by weight of tetraoxyethylene lauryl ether.

4. Composition according to claim 1 characterized in that for tinting the hair at the same time it contains 0.05 to 2.0 % by weight of a dye which is applied directly to the hair.

5. Composition according to claim 4, characterized in that the dye applied directly to the hair is selected from aromatic nitro dyes, azo dyes, anthraquinone dyes and triphenylmethane dyes.

6. Composition according to claims 1 to 5, characterized in that it contains 2 to 10 % by weight of a propellant, which is chosen from n-butane, i-butane, propane, difluorodichloromethane, trichloromonofluoromethane, tetrafluorodichloroethane, dimethyl ether, $N_2$, $N_2O$ and $CO_2$.

## Revendications

1. Produit de fixation de la coiffure et de soins capillaires à base d'une solution dans l'eau, dans l'alcool ou, dans un mélange d'eau et d'alcool, d'un copolymérisat quaternisé de vinylpyrrolidone et de méthacrylate de diméthylaminoéthyle (80 : 20) et d'un alcool gras éthoxylé, ainsi éventuellement que des agents de moussage et des additifs usuels, caractérisé en ce qu'il renferme:

a) 0,1 à 10,0 % en poids d'un copolymérisat quaternisé de vinylpyrrolidone et de méthacrylate de diméthylaminoéthyle (80 : 20), et

b) comme alcool gras éthoxylé, 0,1 à 5,0 % en poids d'éther laurique du tétraoxyéthylène.

2. Produit selon la revendication 1, caractérisé en ce qu'il renferme 1 à 3,5 % en poids d'un produit de copolymérisation, quaternisé avec du sulfate de diméthyle ou de diéthyle, de vinylpyrrolidone et de diméthylaminoéthyle (80 : 20).

3. Produit selon la revendication 1, caractérisé en ce qu'il renferme 0,5 à 2,0 % en poids d'éther laurique de tétraoxyéthylène.

4. Produit selon la revendication 1, caractérisé en ce qu'il renferme, pour teinter en même temps les cheveux 0,05 à 2,0 % en poids d'un colorant prenant directement sur les cheveux.

5. Produit selon la revendication 4, caractérisé en ce que le colorant prenant directement sur les cheveux est choisi parmi des colorants nitrés aromatiques, des colorants azoïques, des colorants anthraquinoniques et des colorants au triphénylméthane.

6. Produit selon les revendications 1 à 5, caractérisé en ce qu'il renferme 2 à 10 % en poids d'un agent de moussage, choisi parmi le n-butane, le propane, l'i-butane, le difluorodichlorométhane, le trichloromonofluorométhane, le tétrafluorodichlorométhane, le diméthyl-éther, $N_2$, $N_2O$ et $CO_2$.